# EUROPEAN PATENT APPLICATION

(11) **EP 2 939 708 A1**
(43) Date of publication of application: **04.11.2015**
(21) Application number: 12891049.4
(22) Date of filing: 26.12.2012
(51) Int. Cl.: A61N 5/10, G01T 1/204, G01T 1/29, G01T 7/00

(54) **DOSE DISTRIBUTION MEASUREMENT DEVICE**

(71) Applicant: Mitsubishi Electric Corporation, Chiyoda-ku Tokyo 100-8310 (JP)
(72) Inventor: HIRASAWA, Kousuke, Tokyo 100-8310 (JP); IKEDA, Masahiro, Tokyo 100-8310 (JP)
(74) Representative: Sajda, Wolf E.
(86) International application number: PCT/JP2012/083641
(87) International publication number: WO 2014/102929

(57) **Abstract**

A dose distribution measurement device includes at least two cameras (7, 8) arranged on a plane perpendicular to the center axis (CA) of irradiation of a water phantom (3) with a particle beam (4) so as to take an image of light emission of a fluorescent substance containing liquid (5) in the water phantom and a dose distribution calculation and evaluation unit (10) having a spot position determination part (12) for determining, from data of the camera image taken with the cameras, the position of a spot irradiated with the particle beam during its staying and a dose addition part (13) for calculating an irradiation dose distribution at the spot position determined by the spot position determination part (12) using a PDD and an OCR stored in a pencil-beam dose-distribution data storage part (16), to add the irradiation dose distribution at each of spot positions.

## Description

### Technical Field

The present invention relates to a dose distribution measurement device for measuring a dose distribution formed by a particle beam that is used in, for example, particle beam therapies for cancers.

### Background Art

In cancer radiation therapy, in order to check the energy and the shape of a therapy radiation beam such as an X-ray, an electron beam, and a particle beam, it is necessary to measure a dose distribution in a water phantom mimicking a human body before irradiating a patient with such a beam.

Furthermore, it is necessary to perform routinely a dose distribution measurement, as quality control on the radiation beam, for adjusting the radiation beam emitting device such as an accelerator and for checking the beam energy distribution and the shape which are different from patient to patient.

For example in Patent Document 1, using a water tank mimicking a human body and an ionization chamber equipped with an actuator for changing the chamber position in the water, a dose distribution formed in the water by irradiation with a radiation beam is measured by scanning the ionization chamber. For that reason, a lot of time and effort is needed for only one dose distribution measurement.

Moreover, since the check by the dose distribution measurement is needed in every change of the beam condition, there is a limit on increase in the number of patients treatable with one irradiation device, i.e., in the availability factor of the therapy apparatus.

In order to overcome such problems, various types of radiation detectors and dose distribution measurement devices have been proposed as devices capable of measuring a dose distribution in a short time.

For example, Patent Document 2 discloses a technology in which a substance that emits fluorescence when excited by radiation is contained in a solid phantom of high visible-light transparency to measure a fluorescence intensity by converting the light emission induced by the radiation into electrical signals using a CCD camera or the like.

Furthermore, Patent Document 3 discloses a particle-beam dose-distribution measurement device including a scintillator unit composed of a scintillation liquid that emits light when irradiated with a proton beam and an imaging unit composed of a CCD camera for taking an image of the scintillator unit in a direction perpendicular to the incident direction of the proton beam, thereby to simultaneously measure scintillations in a plurality of horizontal cross-sectional planes along the incident direction of the particle beam to reconstructed a two-dimensional distribution for each cross-sectional plane and finally to obtain a three-dimensional distribution.

### Prior Art Documents

### Patent Document

Patent Document 1: JP 2003-047 666 A
Patent Document 2: JP 2011-133 598 A
Patent Document 3: JP 2003-079 755 A

### Summary of the Invention

### Problem that the Invention is to Solve

There is a scanning irradiation method in particle beam irradiation methods used in particle beam therapy systems that utilize a so-called particle beam such as a proton beam or a carbon beam among radiation beams. The scanning irradiation method is a method of forming a two-dimensional irradiation distribution perpendicular to the beam traveling direction by shifting a fine particle beam, which is called a pencil beam, in the two-dimensional directions perpendicular thereto.

In addition, since the position where the absorbed dose of the particle beam peaks (referred to as "Bragg peak") depends on the energy of the particle beam, the irradiation position in the beam traveling direction is varied by changing the energy of the particle beam. In the scanning irradiation method, a three-dimensional irradiation field is formed by shifting the pencil beam and changing its energy as described above.

Because the beam irradiation position thus varies with time in the scanning irradiation method, there arise a problem that the technologies disclosed in Patent Documents 1 to 3, which are for measuring a dose distribution when the irradiation position does not vary with time, cannot be directly applied to measurement of a dose distribution formed by the scanning irradiation method, or need a significant time when trying to measure a dose distribution formed by the scanning irradiation method.

The present invention has been made to overcome such an above-described problem, and aims at providing a particle-beam dose-distribution measurement device that is capable of measuring, with a simple configuration and in a short time, a particle beam dose distribution formed by a scanning irradiation method.

### Means for Solving the Problem

The present invention provides a dose distribution measurement device for measuring an irradiation dose distribution to be generated when an irradiation-related device for irradiating an irradiation target with a particle beam as a pencil beam irradiates a three-dimensional target region with the particle beam by scanning, every time an energy level of the particle beam is changed, the particle beam over a two dimensional region of the irradiation target at a depth position corresponding to the energy level with repetition of staying and shifting of the particle beam in a two-dimensional direction perpendicular to the beam traveling direction. The dose distribution measurement device includes:
a water phantom having a fluorescent substance containing liquid that emits light by being irradiated with the particle beam and provided with an incident window for incident of the particle beam;
at least two cameras arranged outside the water phantom and on a plane perpendicular to an irradiation center axis in the water phantom, of the particle beam so as to take images of light emission of the fluorescent substance containing liquid; and
a dose distribution calculation and evaluation unit that includes a camera image processing part processing the images taken with the at least two cameras;
a camera calibration-parameter storage part storing camera calibration parameters for each of the at least two cameras;
a spot position determination part determining a position of a spot irradiated with the particle beam during staying of the particle beam, from camera image data processed by the camera image processing part using the camera calibration parameters for each camera which are stored in the camera calibration-parameter storage part;
a pencil-beam dose-distribution data storage part storing PDD data and OCR data of the pencil beam; and
a dose addition part calculating an irradiation dose distribution at the spot position determined by the spot position determination part using the PDD data and the OCR data stored in the pencil-beam dose-distribution data storage part and adding the irradiation dose distribution at each of spot positions.

### Advantages of the Invention

According to the present invention, a dose distribution measurement device can be provided that is capable of measuring a dose distribution with a simple configuration and in a short time.

### Brief Description of the Drawings

- FIG. 1: is a block diagram schematically showing a configuration of a particle beam irradiation system including a dose distribution measurement device according to Embodiment 1 of the present invention;
- FIG. 2: shows graphs showing examples of a PDD and an OCR of a pencil beam;
- FIG. 3: is a chart for explaining spot positions determined from a camera image obtained by the dose distribution measurement device according to Embodiment 1 of the present invention;
- FIG. 4: is a flow diagram showing an operation of the dose distribution measurement device according to Embodiment 1 of the present invention;
- FIG. 5: is a graph showing an example of a two-and-half--dimensional dose distribution obtained by the dose distribution measurement device according to Embodiment 1 of the present invention;
- FIG. 6: is a block diagram schematically showing a configuration of a particle beam irradiation system including a dose distribution measurement device according to Embodiment 2 of the present invention;
- FIG. 7: is a block diagram schematically showing a configuration of a particle beam irradiation system including a dose distribution measurement device according to Embodiment 3 of the present invention;
- FIG. 8: is a flow diagram showing an operation of the dose distribution measurement device according to Embodiment 3 of the present invention;
- FIG. 9: is a block diagram schematically showing a configuration of a particle beam irradiation system including a dose distribution measurement device according to Embodiment 4 of the present invention;
- FIG. 10: is a flow diagram showing an operation of the dose distribution measurement device according to Embodiment 4 of the present invention;
- FIG. 11: is a chart showing an example of a camera image obtained by the dose distribution measurement device according to Embodiment 4 of the present invention;
- FIG. 12: is a graph showing an example of a one-dimensional light intensity distribution extracted from a camera image obtained by the dose distribution measurement device according to Embodiment 4 of the present invention;
- FIG. 13: is a graph showing an example of another one-dimensional light intensity distribution extracted from a camera image obtained by the dose distribution measurement device according to Embodiment 4 of the present invention;
- FIG. 14: is a flow diagram showing an operation of a dose distribution measurement device according to Embodiment 5 of the present invention; and
- FIG. 15: is a table for explaining an effect of the present invention.

### Embodiments for Carrying Out the Invention

### Embodiment 1

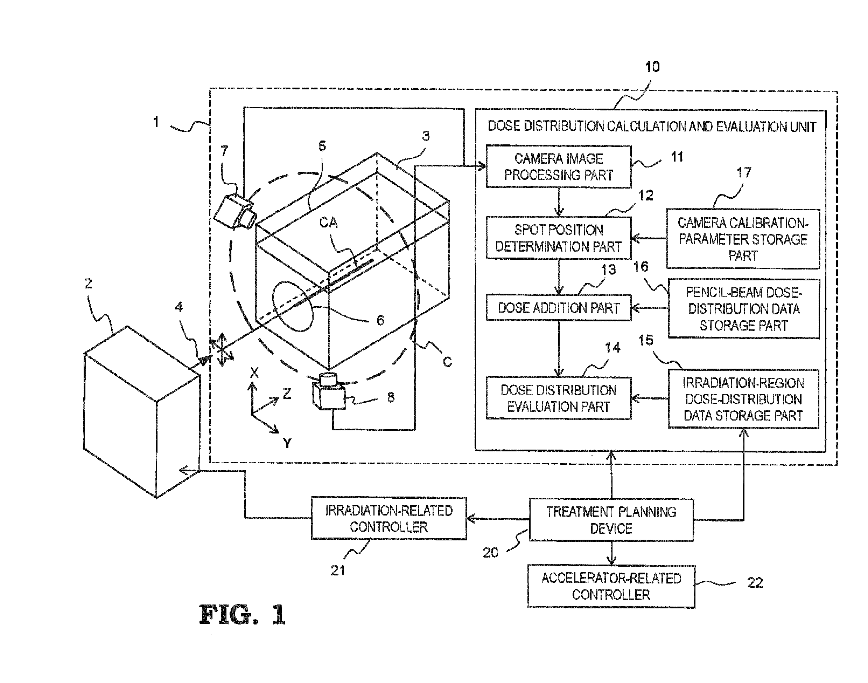

FIG. 1 is a block diagram schematically showing a configuration of a particle beam irradiation system including a dose distribution measurement device 1 according to Embodiment 1 of the present invention. A particle beam 4 is emitted from an irradiation-related device 2 toward a water phantom 3 constituted with a water tank.

The water phantom 3 is filled with a fluorescent substance containing liquid 5 (generally referred to as a liquid scintillator) that emits light on absorbing the particle beam. The wall of the water phantom 3 is formed of a transparent material such as an acrylic resin that transmits light. At a portion where the particle beam 4 is incident, there is provided an incident window 6 made of a similar material such as an acrylic resin that little absorbs the particle beam.

For little absorption of the particle beam, the incident window 6 is sometimes formed thinner than the other portion. Two cameras of a camera 7 and a camera 8 are provided around the water phantom 3. The two cameras are arranged, for example, on a circle C, which is in a plane perpendicular to the irradiation center axis CA of the particle beam 4 and centered on the irradiation center axis CA, to take an image whose center is on the irradiation center axis CA.

A dose distribution calculation and evaluation unit 10 calculates and evaluates a dose distribution from the camera images taken with the cameras 7 and 8. The dose distribution calculation and evaluation unit 10 includes a camera image processing part 11 for processing the camera images; a spot position determination part 12 for determining a spot position from a camera image processed by the camera image processing part 11 using camera calibration parameters stored in a camera calibration-parameter storage part 17.

The dose distribution calculation and evaluation unit 10 further includes a dose addition part 13 for calculating and adding a dose at each of spot positions using a pencil beam dose distribution stored in a pencil-beam dose-distribution data storage part 16; and a dose distribution evaluation part 14 for comparing a measured dose distribution that is a result added by the dose addition part 13 with irradiation-region dose distribution data that is planned in a treatment planning device 20 and stored in an irradiation-region dose-distribution data storage part 15 for storing the irradiation-region dose distribution data, to evaluate the measured dose distribution.

A particle beam 4, which is called a fine pencil beam, is shifted in the two-dimensional directions perpendicular to the beam traveling direction by the irradiation-related device 2 to form a two-dimensional irradiation distribution perpendicular to the beam traveling direction.

Here, the beam traveling direction is defined as a Z-direction and two directions perpendicular to the Z-direction, i.e., directions in which the beam is shifted are defined as an X-direction and a Y-direction. The irradiation-related device 2 includes an X-direction deflection electromagnet and a Y-direction deflection electromagnet for deflecting the particle beam 4 in the X-direction and the Y-direction, respectively.

The particle beam 4 is emitted with repetition of shifting and staying by the irradiation-related device 2. That is, the particle beam 4 stays at an irradiation position (hereinafter referred to as a spot position) and when the irradiation dose at the spot position reaches a planned irradiation dose, the particle beam 4 is shifted to the next spot position and is emitted until the irradiation dose at the next spot position reaches its planned irradiation dose.

Repetition of the shifting and staying for an energy level of the particle beam 4 forms a planned irradiation dose distribution, i.e., a two-dimensional irradiation dose distribution in an irradiation region at a Bragg peak position corresponding to the energy level, i.e., at a depth position in the beam traveling direction. Since the irradiation depth is varied by changing the energy level of the particle beam, the energy level of the particle beam is changed to form a planned two-dimensional irradiation dose distribution in an irradiation region at another depth position.

In this way, a planned irradiation dose distribution is finally formed in a three-dimensional irradiation region by forming two-dimensional irradiation dose distributions with repetition of shifting and staying of the particle beam 4 for each different energy level. Such an irradiation method described above is here referred to as a spot scanning irradiation method.

In order to form an irradiation dose distribution in an irradiation region needed for a diseased part of a patient using the above spot scanning irradiation method, the treatment planning device 20 calculates control parameters for an irradiation-related controller 21 to control the irradiation-related device 2 and control parameters for an accelerator-related controller 22 to control a not-shown accelerator, and transmits these control parameters to the irradiation-related controller 21 and the accelerator-related controller 22.

While the diseased part of the patient is irradiated with the particle beam 4 with repetition of shifting and staying in accordance with these control parameters for the irradiation- related controller 21 and the accelerator-related controller 22 during the therapy, a dose distribution in the diseased part of the patient cannot be directly measured.

For that reason, the dose distribution measurement device 1 is used in advance to the therapy in order to check whether the planned dose distribution is formed when the irradiation is performed in accordance with these control parameters.

A dose distribution measuring method using the dose distribution measurement device 1 according to Embodiment 1 of the present invention will be described with reference to FIGS. 1 to 5. Dose distribution characteristics of a pencil beam are measured first before a three-dimensional dose distribution is measured by the dose distribution measurement device 1.

The dose distribution characteristics of the pencil beam is measured as a percent depth dose (PDD), which is a distribution in the Z-direction, and as an off center axis ratio (OCR), which is a distribution in the X-Y plane. The OCR and the PDD can be measured with instruments such as a heretofore known thimble dosimeter and an Advanced Markus®, respectively, or also measured by a method later-described in Embodiment 5.

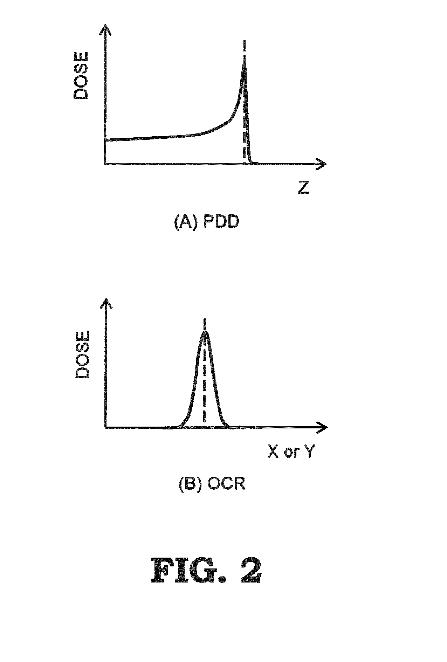

Examples of a PDD and an OCR are respectively shown in the graphs (A) and (B) of FIG. 2. These PDD and OCR are measured for each energy level of the particle beam, and stored in the pencil-beam dose- distribution data storage part 16 of the dose distribution calculation and evaluation unit 10.

Next, with the configuration of FIG. 1, the water phantom 3 is irradiated with the particle beam 4 by the spot scanning irradiation method. Specifically, each spot is irradiated with the particle beam 4 by controlling the accelerator and the irradiation-related device 2 in accordance with the control parameters for each spot position, of the accelerator-related controller 22 and the irradiation-related controller 21.

Every time the energy level of the particle beam 4 is changed, the particle beam 4 is scanned with repetition of staying and shifting in the two-dimensional directions perpendicular to the traveling direction over a two-dimensional irradiation region in the water phantom at a depth position corresponding to the energy level.

A three-dimensional irradiation region is thereby irradiated with the particle beam 4. At this time, an image of light emission of the fluorescent substance containing liquid 5 is taken for each irradiation spot position with the cameras 7 and 8. The highest intensity point in the taken image is extracted for each spot position.

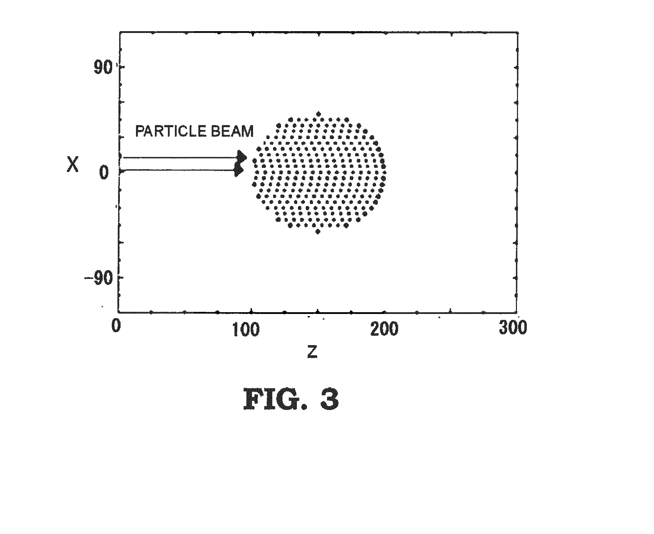

For example, plotting highest intensity points at all spot positions by extracting the highest intensity point in the image taken for each spot position with the camera 7, data as shown in FIG. 3 is obtained.

A three-dimensional dose distribution that is obtained by the spot irradiation is calculated for each spot position using the PDD and the OCR data stored in the pencil-beam dose- distribution data storage part 16. An integrated three-dimensional irradiation distribution can be obtained by integrating the three-dimensional dose distributions at all spot positions.

In order to three-dimensionally measure a three-dimensional position by cameras in two or more directions, it is necessary to perform calibration for the cameras in advance. To be more specific, external camera parameters (mounting position and angle) and internal camera parameters (such as an image center and distortion), which are calibration parameters of the cameras, can be determined by a known method.

Here, a calibration point whose three-dimensional coordinate position is given is implanted in the water phantom so that the calibration point can be positioned at the isocenter of a therapeutic coordinate using a laser pointer or the like in the treatment room. The external and internal parameters of each camera are calculated using the calibration point in each camera image. The calculated external and internal calibration parameters of each camera are stored in the camera calibration-parameter storage part 17.

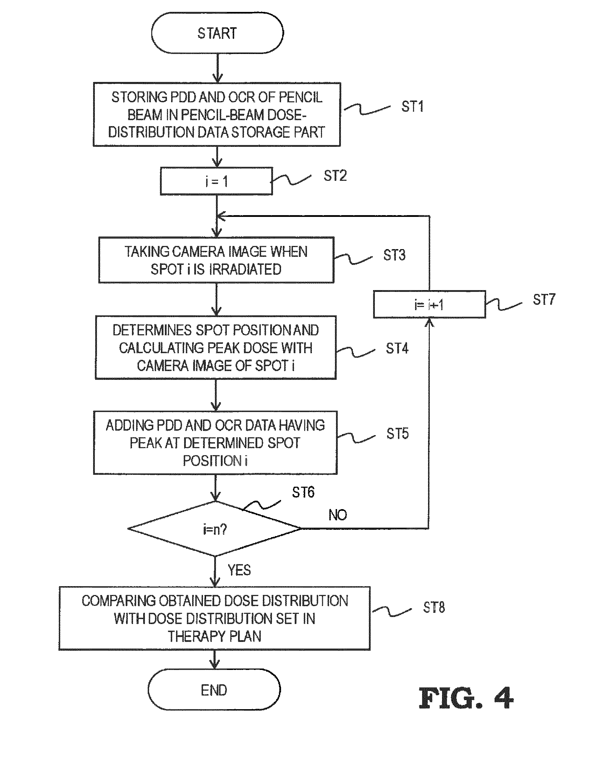

The above measurement method is explained with reference to the flow diagram of FIG. 4. As described above, the PDD and the OCR of the pencil beam are measured in advance for each particle-beam energy level, to be stored in the pencil-beam dose-distribution data storage part 16 of the dose distribution calculation and evaluation unit 10 (Step ST1).

Here, the number of positions of the spots to be irradiated is assumed to be n. Firstly, i is set to be one (i = 1) to obtain measured irradiation dose distribution data at the first spot position (Step ST2). The control parameters of the irradiation-related controller 21 and the accelerator-related controller 22 are set for the first position spot to be irradiated, and then images of light emission of the water phantom 3 are taken with the cameras 7 and 8 when the spot I = 1 is irradiated (Step ST3).

From camera images taken with the cameras 7 and 8, the spot position determination part 12 determines the highest intensity position as a three-dimensional position of the spot and calculates a peak dose from the intensity (Step ST4). In determining the three-dimensional position, the camera calibration parameters of each camera stored in the camera calibration-parameter storage part 17 are used.

In addition, in a case of the intensity and the absorbed dose being in a nonlinear relationship, tabulating the nonlinear relationship between the intensity and the absorbed dose in a correspondence table would allow for simple conversion from the intensity to the absorbed dose.

The dose addition part 13 calculates an irradiation dose distribution that has the peak at the determined spot position I = 1 using the PDD and the OCR data stored in the pencil-beam dose-distribution data storage part, to obtain a three-dimensional irradiation dose distribution (Step ST5).

Next, the increment of i = i + 1 is performed (Step ST7), i.e., i is increased to two (i = 2), and then the control parameters of the irradiation-related controller 21 and the accelerator-related controller 22 are set for the second position spot to be irradiated.

And images of light emission of the water phantom 3 are taken with the cameras 7 and 8 when the spot i = 2 is irradiated (Step ST3). From camera images taken with the cameras 7 and 8, the spot position determination part 12 determines the highest intensity position as a three-dimensional position of the spot and calculates the peak dose from the intensity (Step ST4).

An irradiation dose distribution that has a peak at the determined peak spot position i =2 is calculated using the PDD and the OCR data stored in the pencil-beam dose-distribution data storage part, to be added to the first three-dimensional irradiation dose distribution (Step ST5).

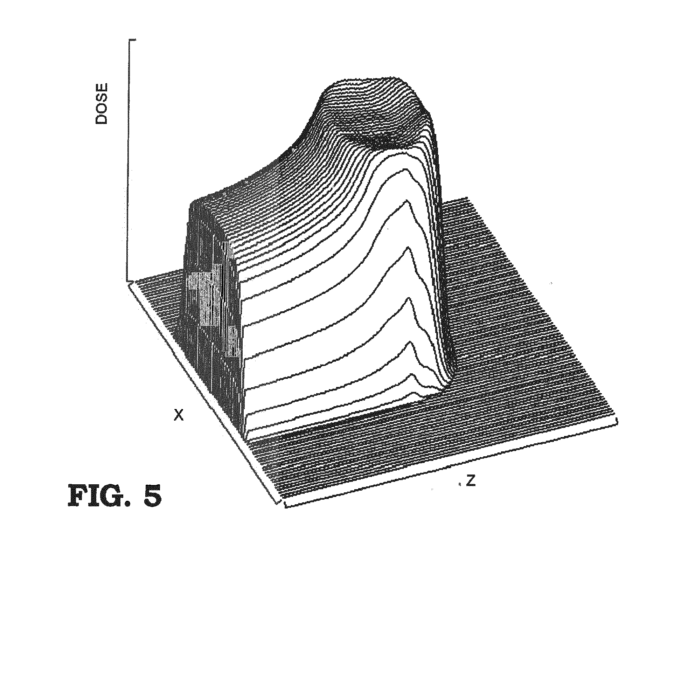

In this way, calculated three-dimensional irradiation dose distributions are added until i becomes i = n ("NO" in ST6). At the time of finishing irradiation at all spot positions, i.e., i = n ("YES" in ST6), an irradiation dose distribution obtained by the addition is a measured irradiation dose distribution. An example of obtained data is shown in FIG. 5.

FIG. 5 is a graph showing a two-dimensional distribution in the X- and the Z-directions at a Y-position near the center, that is, a distribution chart of two-and-half--dimensions. In practice, a three-dimensional irradiation dose distribution such as having point-by-point values in the X, Y, Z three-dimensional volume is obtained. The dose distribution evaluation part 14 compares the measured three-dimensional irradiation dose distribution thus obtained with a three-dimensional irradiation dose distribution set in a treatment plan and evaluates the measured dose distribution (Step ST8).

The dose distribution set in the treatment plan is stored in advance in the irradiation-region dose-distribution data storage part 15 of the dose distribution calculation and evaluation unit 10 from the treatment planning device. Evaluation to what degree the measured irradiation dose distribution conforms to the irradiation dose distribution set in the treatment plan can be performed using a known index such as a gamma index.

As described above, the dose distribution measurement device according to Embodiment 1 of the present invention is capable of easily measuring a dose distribution of a spot scanning irradiation by determining spot positions from an image obtained by taking light emission of the water phantom 3 induced by irradiation of each spot with the two cameras 7 and 8 arranged on the circle C centered on the irradiation center axis CA and using the in advance measured PDD and OCR data of a pencil beam. It is desirable to arrange the two cameras at positions for their imaging angles to be perpendicular to each other.

It should be noted that the two cameras are not necessarily arranged at the perpendicular positions because determination of camera calibration parameters corresponding to the camera arrangement permits a spot position to be determined from camera images. In addition, it is sufficient to arrange at least two cameras; three or more cameras may as well be arranged. More cameras allows for determination of a spot position with higher accuracy.

### Embodiment 2

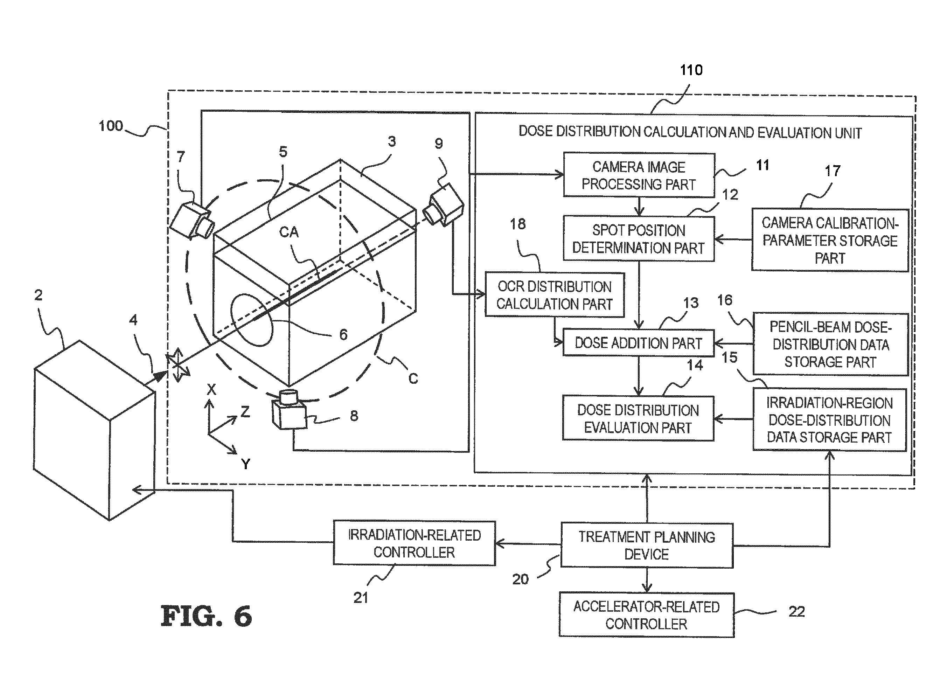

FIG. 6 is a block diagram schematically showing a configuration of a particle beam irradiation system that includes a dose distribution measurement device 100 according to Embodiment 2 of the present invention. In FIG. 6, the same reference numerals as those in FIG. 1 designate the same or equivalent components.

In the dose distribution measurement device 100 according to Embodiment 2, an OCR measuring camera 9 is added to the configuration of the dose distribution measurement device 1 of Embodiment 1. The OCR measuring camera 9 is disposed outside the water phantom 3 and toward the opposite side of the incident window 6 of the water phantom 3 to take an image toward the water phantom 3. The image is taken every time a spot is irradiated, thus obtaining an image corresponding to the OCR for each spot irradiation.

An OCR distribution calculation part 18 in a dose distribution calculation and evaluation unit 110 calculates OCR data for each spot irradiation from these images. In calculating the OCR data, the beam diameter is calculated from the camera images and the beam distribution may be assumed to have, for example, a Gaussian distribution.

In Embodiment 2, the dose addition part 13 calculates and adds, in the step ST5 described in Embodiment 1, an irradiation dose distribution having a peak at the spot position i determined by the spot position determination part 12 using the OCR data calculated by the OCR distribution calculation part 18 and the PDD data stored in the pencil-beam dose-distribution data storage part 16.

As described above, according to Embodiment 2, there is no need to measure and store in advance the OCR of the pencil beam, and an OCR at the time of actual irradiation is measured to be used for calculation of a dose distribution, thus measuring a dose distribution with higher accuracy.

### Embodiment 3

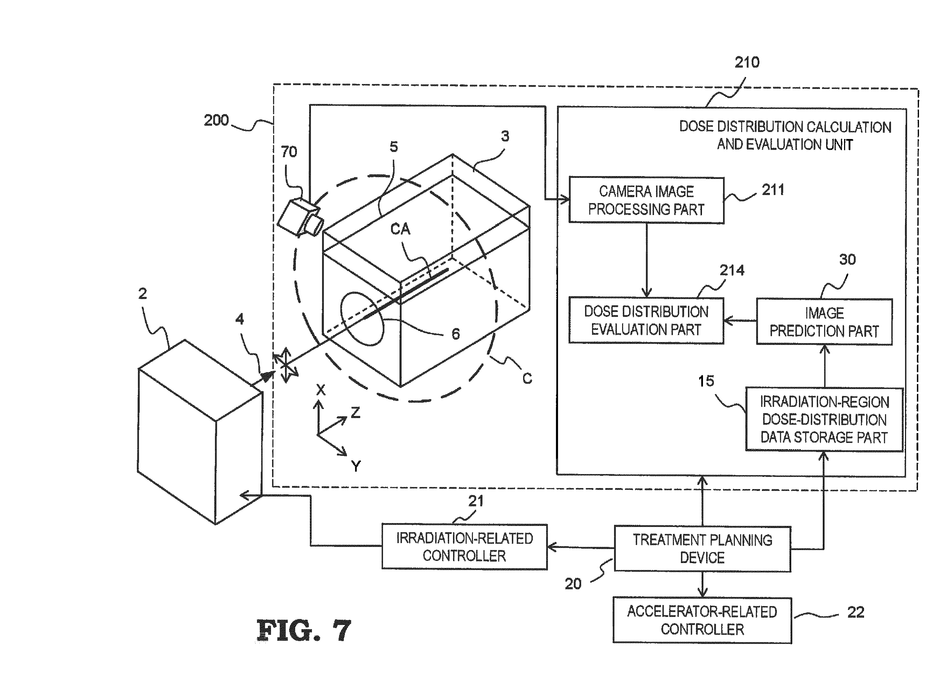

FIG. 7 is a block diagram schematically showing a configuration of a particle beam irradiation system that includes a dose distribution measurement device 200 according to Embodiment 3 of the present invention. In FIG. 7, the same reference numerals as those in FIG. 1 designate the same or equivalent components.

In Embodiment 3, one camera 70 is provided outside the water phantom 3 and disposed, for example, on a circle C, which is in a plane perpendicular to the irradiation center axis CA of the particle beam 4 and centered on the irradiation center axis CA, to take an image whose center is on the irradiation center axis CA.

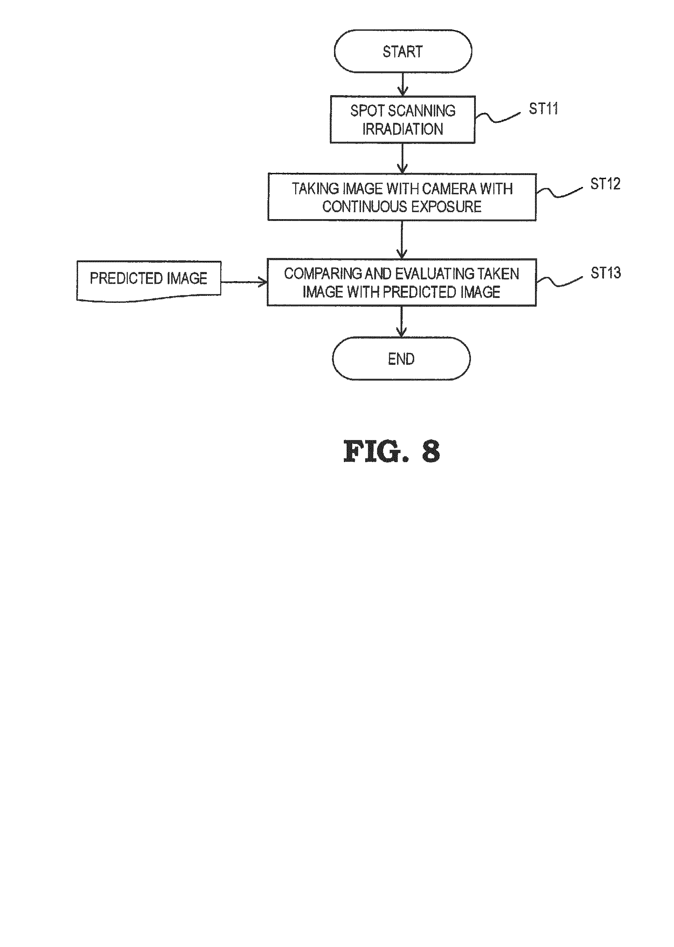

FIG. 8 shows an operational flow of the dose distribution measurement device 200 according to Embodiment 3. The water phantom 3 is irradiated with the particle beam 4 by the spot scanning irradiation method (Step ST11) and then an image of light emission of the fluorescent substance containing liquid 5 is taken with the camera 70 with a continuous exposure during the irradiation (Step ST12).

The light emission is recorded as integrated values in a camera image processing part 211 in a dose distribution calculation and evaluation unit 210. The image to be recorded may be an image that expresses the light intensity by a grey scale or by iso-intensity curves that connect points of the same light intensity. The recorded image is not the dose distribution itself but is values integrated temporally and spatially in the optical axis direction of the camera.

Hence, an image prediction part 30 simulates light emission amounts of the water phantom 3 induced by the dose distribution using irradiation-region dose distribution data set in the treatment planning device 20 and stored in the irradiation-region dose-distribution data storage part 15, and predicts an image as spatially and temporally integrated values that would be taken at the position of the camera 70 from the simulated light emission amounts, to stores the predicted image. Since the light emission of the fluorescent substance containing liquid 5 is almost nonlinear to an irradiation dose, it is preferable to take the nonlinearity into account in the simulation.

A dose distribution evaluation part 214 compares the dose distribution at the time of actual irradiation with the dose distribution set in the treatment planning device 20 and evaluates the actual dose distribution by comparing the taken image recorded in the camera image processing part 211 with the predicted image stored in the image prediction part 30 and evaluating the taken image (Step ST13).

As described above, the dose distribution measurement device according to Embodiment 3 is not capable of measuring directly a dose distribution itself, but is capable of comparing with the one camera configuration an actual irradiation dose distribution with a dose distribution set in the treatment planning device with the one camera configuration, thereby evaluating indirectly the actual irradiation dose distribution.

### Embodiment 4

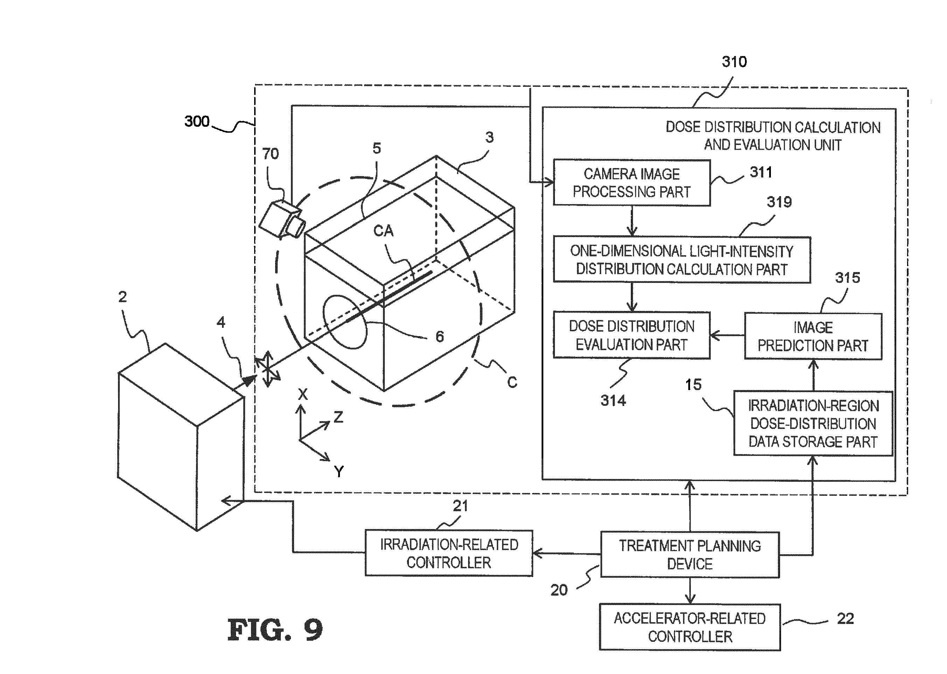

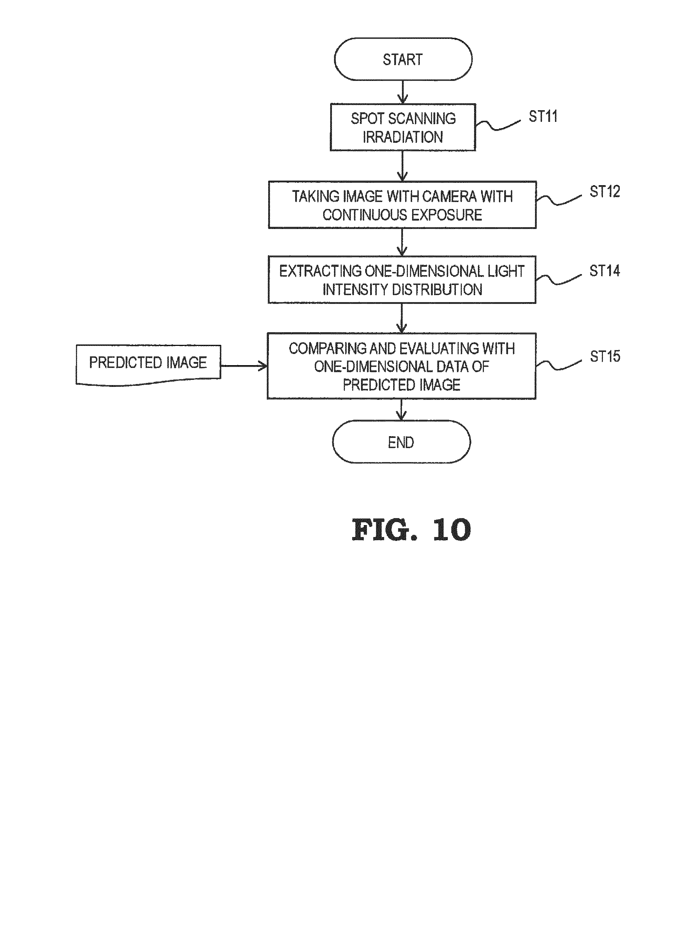

FIG. 9 is a block diagram schematically showing a configuration of a particle beam irradiation system that includes a dose distribution measurement device 300 according to Embodiment 4 of the present invention. And FIG. 10 is a flow diagram showing an operation of the dose distribution measurement device according to Embodiment 4. In FIG. 9, the same reference numerals as those in FIG. 7 designate the same or equivalent components.

In Embodiment 4, the one camera 70 is provided outside the water phantom 3 and disposed, for example, on a circle C, which is in a plane perpendicular to the irradiation center axis CA of the particle beam 4 and centered on the irradiation center axis CA, to take an image whose center is on the irradiation center axis CA, as with Embodiment 3.

In Embodiment 4, similarly to Embodiment 3, the water phantom 3 is irradiated with the particle beam 4 by the spot scanning irradiation method (Step ST11) and then an image of light emission of the fluorescent substance containing liquid 5 is taken with the camera 70 with a continuous exposure during the irradiation (Step ST12), thereby to record the light emission as as integrated values in a camera image processing part 311 in a dose distribution calculation and evaluation unit 310. The recorded image is not the dose distribution itself but is the values integrated temporally and spatially in the optical axis direction of the camera.

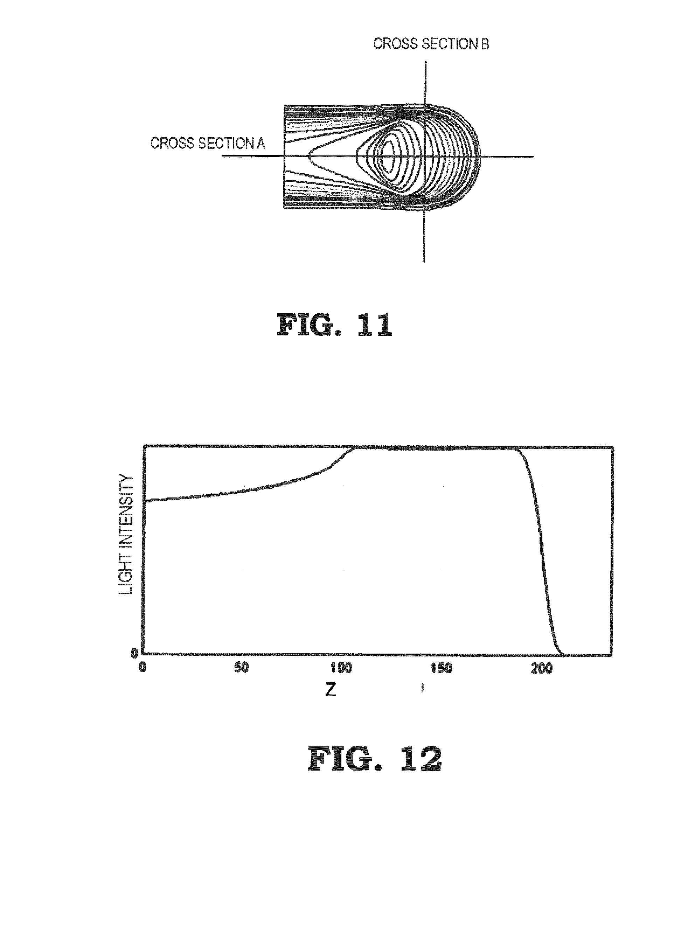

The image is recorded as data expressing, for example, iso-curves of the light intensity as FIG. 11. A one-dimensional light-intensity distribution calculation part 319 of the dose distribution calculation and evaluation unit 310 extracts from the image a one-dimensional light intensity distribution in a cross section A parallel to the irradiation center axis CA and a one-dimensional light intensity distribution in a cross section B perpendicular to the irradiation center axis CA (Step ST14).

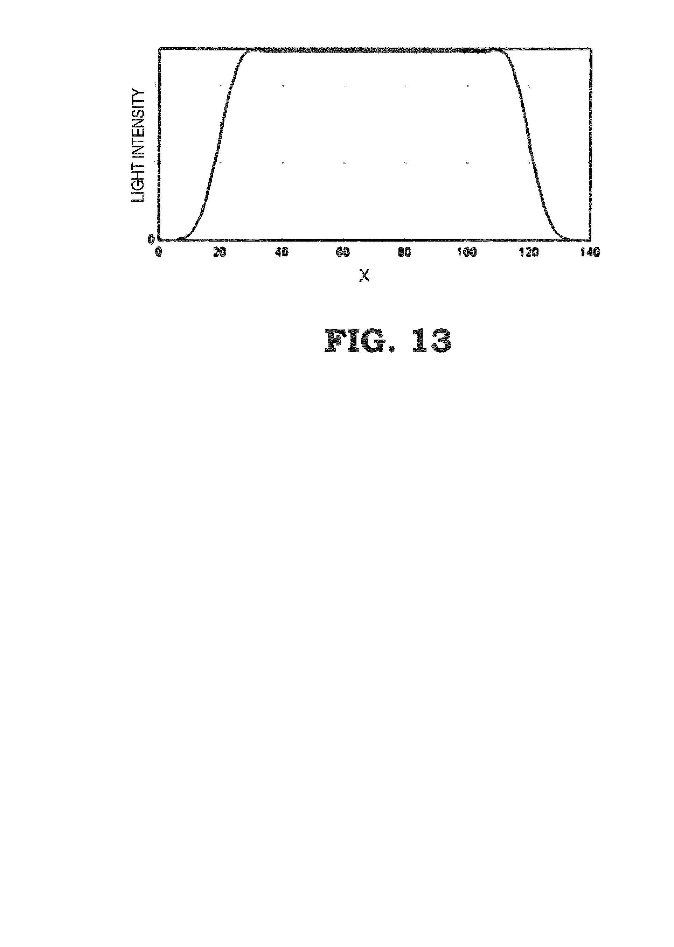

Examples of the extracted light intensity distributions are shown in FIGS. 12 and 13. FIG. 12 is an example of a one-dimensional, i.e., Z-directional light intensity distribution in the cross section A parallel to the irradiation center axis CA, and FIG. 13 is an example of a one-dimensional, i.e., X-directional light intensity distribution in the cross section B perpendicular the irradiation center axis CA.

Meanwhile, an image prediction part 315 simulates light emission amounts of the water phantom 3 induced by the dose distribution using irradiation-region dose distribution data set in the treatment planning device 20 and stored in the irradiation-region dose-distribution data storage part 15, and predicts an image as spatially and temporally integrated values that would be taken at the position of the camera 70 from the simulated light emission amounts, to extract, from the predicted image, one-dimensional light intensity distributions in a cross section A parallel to the irradiation center axis CA and in a cross section B perpendicular to the irradiation center axis CA.

The dose distribution evaluation part 314 evaluates the irradiation dose distribution by comparing the one-dimensional light intensity distributions extracted, from the taken image, by the one-dimensional light intensity distribution calculation part 319 with the one-dimensional light intensity distributions extracted, from the predicted image, by the image prediction part 315 (Step ST15).

### Embodiment 5

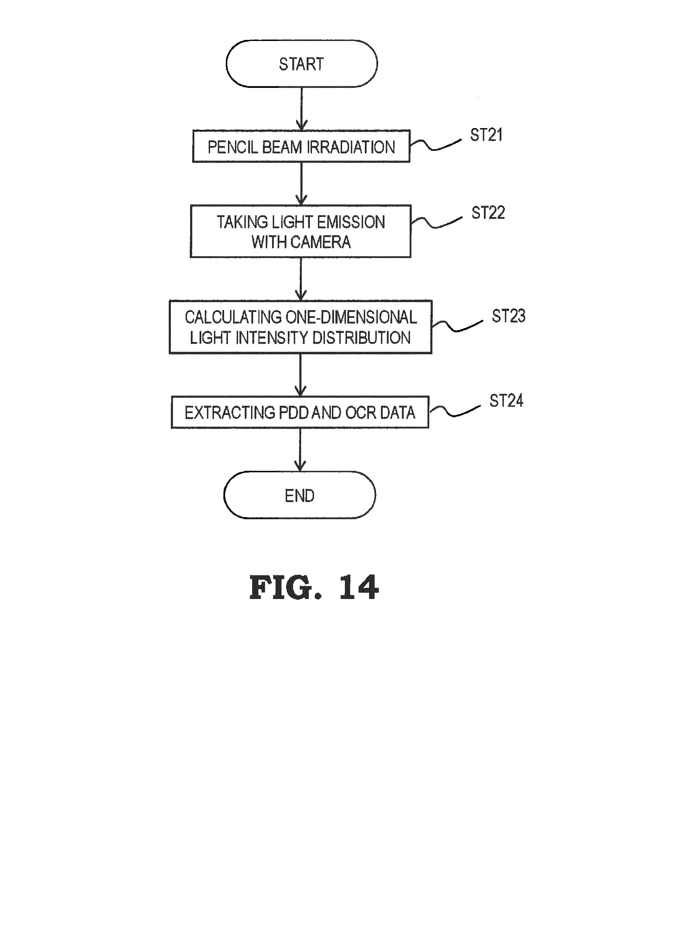

FIG. 14 is an operational flow diagram of a dose distribution measurement device according to Embodiment 5 of the present invention. The dose distribution measurement device according to Embodiment 5 can obtain pencil beam source data of the particle beam 4 with the configuration of the dose distribution measurement device 300 shown in FIG. 9, which is the same configuration as Embodiment 4, by irradiating the water phantom 3 for a short time without shifting the particle beam 4 as the pencil beam (Step ST21) and then by taking an image of light emission induced by the pencil beam with the camera 70 (Step ST22).

When the camera 70 is disposed as shown in FIG.9, for example, one-dimensional light intensity distribution in the Z-direction and a one-dimensional light intensity distribution in the X-direction calculated by a one-dimensional light intensity distribution calculation part 319 (Step ST23) are a distribution corresponding to a PDD and a distribution corresponding to an X-directional OCR, respectively, of the source beam.

Hence, the dose distribution evaluation part 314 extract the PDD and the OCR data of the source beam from one-dimensional intensity distributions calculated by the one-dimensional light intensity distribution calculation part 319 (Step ST24).

When one more camera is further disposed in a direction perpendicular to the camera 70, i.e., at the position of the camera 8 shown in FIG.1, a distribution corresponding to a Y-directional OCR of the source beam can be obtained. In a case of the intensity and the absorbed dose being in a nonlinear relationship, tabulating the nonlinear relationship therebetween as a correspondence table allows for easy conversion from the light intensity to the absorbed dose.

In this way, by taking with a camera an image of light emission of the liquid containing a fluorescent substance induced by the short irradiation with the pencil beam allows for obtaining easily source beam data corresponding to the PDD and the OCR of the pencil beam.

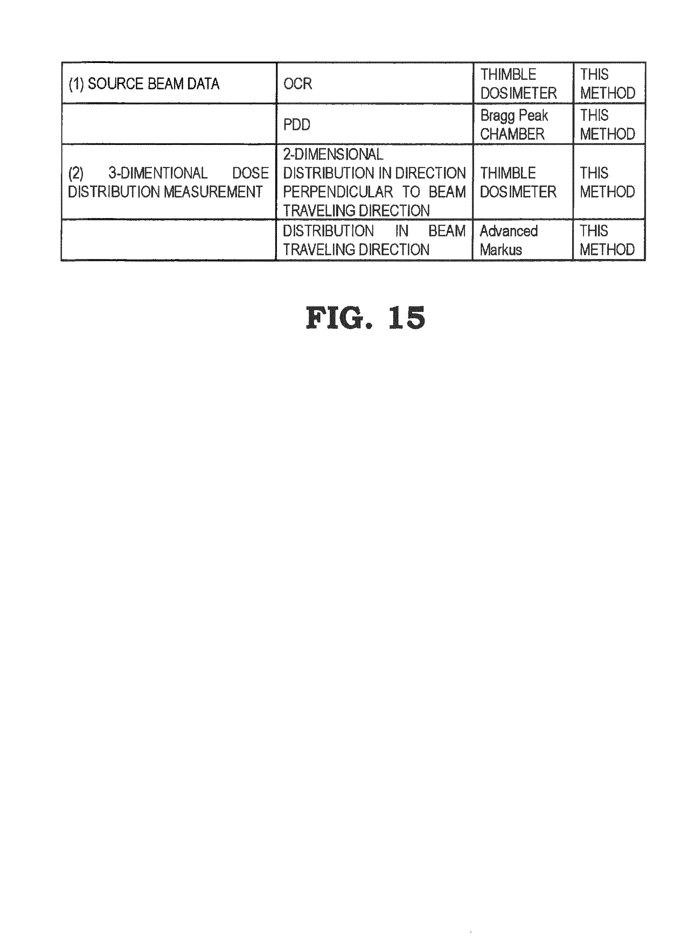

From the object of particle beam therapy, two situations and two measurements, i.e., four cases in total are conceivable as shown in FIG. 15. The first situation is measurements for registering source beam data for a treatment plan. In the measurement, a thimble dosimeter or the like is used for the OCR measurement.

Also in the measurement, a Bragg Peak chamber or the like is used for the PDD measurement. The other application (situation) is distribution measurements for validating in advance whether an irradiation dose of a patient is in accordance with that simulated in the treatment plan.

In the distribution measurements, the OCR is often measured with a thimble dosimeter or the like, and the PDD is often measured with an Advanced Markus® or the like.

Employing each technique according to the present invention allows for covering all the four cases, thus exhibiting the feature of performing two-dimensional, two-and-half-dimensional, or three-dimensional distribution measurement.

In the present invention, each Embodiment may be freely combined and/or appropriately modified and/or omitted within the scope and spirit of the invention.

### List of Reference Numerals

- 1: dose distribution measurement device
- 2: irradiation-related device
- 3: water phantom
- 4: particle beam
- 5: fluorescent substance containing liquid
- 6: incident window
- 7: camera
- 8: camera
- 9: OCR measuring camera
- 10: dose distribution calculation and evaluation unit
- 11: camera image processing part
- 12: spot position determination part
- 13: dose addition part
- 14: dose distribution evaluation part
- 15: irradiation-region dose-distribution data storage part
- 16: pencil-beam dose-distribution data storage part
- 17: camera calibration-parameter storage part
- 18: OCR distribution calculation part
- 20: treatment planning device
- 21: irradiation-related controller
- 22: accelerator-related controller 22
- 30: image prediction part
- 70: camera
- 100: dose distribution measurement device
- 110: dose distribution calculation and evaluation unit
- 200: dose distribution measurement device
- 210: dose distribution calculation and evaluation unit
- 211: camera image processing part
- 214: dose distribution evaluation part
- 300: dose distribution measurement device
- 310: dose distribution calculation and evaluation unit
- 311: camera image processing part
- 314: dose distribution evaluation part
- 315: image prediction part
- 319: one-dimensional light intensity distribution calculation part.

## Claims

1. A dose distribution measurement device for measuring an irradiation dose distribution to be generated when an irradiation-related device for irradiating an irradiation target with a particle beam as a pencil beam irradiates a three-dimensional target region with the particle beam by scanning, every time an energy level of the particle beam is changed, the particle beam over a two dimensional region of the irradiation target at a depth position corresponding to the energy level with repetition of staying and shifting of the particle beam in a two-dimensional direction perpendicular to the beam traveling direction,
the dose distribution measurement device comprising:
- a water phantom having a fluorescent substance containing liquid that emits light by being irradiated with the particle beam and provided with an incident window for incident of the particle beam;
- at least two cameras arranged outside the water phantom and on a plane perpendicular to an irradiation center axis in the water phantom, of the particle beam so as to take images of light emission of the fluorescent substance containing liquid; and
- a dose distribution calculation and evaluation unit including:
- a camera image processing part processing the images taken with the at least two cameras;
- a camera calibration-parameter storage part storing camera calibration parameters for each of the at least two cameras;
- a spot position determination part determining a position of a spot irradiated with the particle beam during staying of the particle beam, from camera image data processed by the camera image processing part using the camera calibration parameters for each camera which are stored in the camera calibration-parameter storage part;
- a pencil-beam dose-distribution data storage part storing PDD data and OCR data of the pencil beam; and
- a dose addition part calculating an irradiation dose distribution at the spot position determined by the spot position determination part using the PDD data and the OCR data stored in the pencil-beam dose-distribution data storage part and adding the irradiation dose distribution at each of spot positions.

2. The dose distribution measurement device set forth in claim 1 further comprising:
an OCR measuring camera provided outside the water phantom and on a side opposite the incident side of the particle beam,
wherein the dose distribution calculation and evaluation unit further includes an OCR distribution calculation part calculating an OCR at each spot position from an image taken with the OCR measuring camera and
wherein the dose addition part calculates an irradiation dose distribution at a spot position determined by the spot position determination part using the OCR calculated by the OCR distribution calculation part instead of the OCR data stored in the pencil-beam dose-distribution data storage part.

3. The dose distribution measurement device set forth in claim 1 or 2, wherein the dose distribution calculation and evaluation unit further includes an irradiation-region dose-distribution data storage part storing a dose distribution data for an irradiation region set in a treatment planning device, and compares a measured irradiation dose distribution obtained by adding all irradiation dose distributions at determined spot positions by the dose addition part with the dose distribution data stored in the irradiation-region dose-distribution data storage part, thereby to evaluate the measured irradiation dose distribution.

4. A dose distribution measurement device for measuring an irradiation dose distribution to be generated when an irradiation-related device for irradiating an irradiation target with a particle beam as a pencil beam irradiates a three-dimensional target region with the particle beam by scanning, every time an energy level of the particle beam is changed, the particle beam over a two dimensional region of the irradiation target at a depth position corresponding to the energy level with repetition of staying and shifting of the particle beam in a two-dimensional direction perpendicular to the beam traveling direction,
he dose distribution measurement device comprising:
- a water phantom having a fluorescent substance containing liquid that emits light by being irradiated with the particle beam and provided with an incident window for incident of the particle beam;
- one camera disposed outside the water phantom and on a plane perpendicular to an irradiation center axis in the water phantom, of the particle beam so as to take images of light emission of the fluorescent substance containing liquid; and
- a dose distribution calculation and evaluation unit including:
- a camera image processing part processing an image taken with the one camera;
- an irradiation-region dose-distribution data storage part storing a dose distribution data for an irradiation region set in a treatment planning device;
- an image prediction part predicting a camera image taken at the position of the one camera from the dose distribution data stored in the irradiation-region dose-distribution data storage part, to store the predicted image; and
- a dose distribution evaluation part evaluating the one camera taken image processed by the camera image processing part with the predicted image stored in the image prediction part, to evaluate the one camera taken image.

5. The dose distribution measurement device set forth in claim 4,
wherein the dose distribution calculation and evaluation unit further includes a one-dimensional light intensity distribution calculation part extracting a one-dimensional light intensity distribution from the camera image processed by the camera image processing part, and wherein the dose distribution evaluation part extracts a one-dimensional light intensity distribution from the predicted image stored in the image prediction part and compares the one-dimensional light intensity distribution calculated by the one-dimensional light intensity distribution calculation part with the one-dimensional light intensity distribution extracted from the predicted image thereby to evaluate the calculated one-dimensional light intensity distribution.

6. A dose distribution measurement device that measures pencil beam source data of a particle beam,
the dose distribution measurement device comprising:
- a water phantom having a fluorescent substance containing liquid that emits light by being irradiated with the particle beam and provided with an incident window for incident of the particle beam;
- one camera disposed around the water phantom and on a plane perpendicular to an irradiation center axis in the water phantom, of the particle beam so as to take images of light emission of the fluorescent substance containing liquid; and
- a dose distribution calculation and evaluation unit including:
- a camera image processing part processing an image taken with the one camera;
- a one-dimensional light intensity distribution calculation part extracting a one-dimensional light intensity distribution from an image processed by the camera image processing part from a camera image, taken with the one camera, of light emission induced by irradiation of the fluorescent substance containing liquid with the particle beam as the pencil beam during staying of the particle beam;
- a dose distribution evaluation part obtaining PDD data and OCR data of the particle beam as the pencil beam from the one-dimensional light intensity distribution extracted by the one-dimensional light intensity distribution calculation part.
